# EUROPEAN PATENT APPLICATION

(11) **EP 4 737 064 A1**
(43) Date of publication of application: **06.05.2026**
(21) Application number: 23945150.3
(22) Date of filing: 13.07.2023
(51) Int. Cl.: B25J 3/00, B25J 13/02, G05G 1/00, G05G 1/04

(54) **CONSOLE DEVICE**

(71) Applicant: Riverfield Inc., Tokyo 107-0052 (JP)
(72) Inventor: SAIGA, Shohei, Tokyo 107-0052 (JP)
(74) Representative: Adares PartGmbB
(86) International application number: PCT/JP2023/025847
(87) International publication number: WO 2025/013277

(57) **Abstract**

The purpose of one or more embodiments of the present disclosure is to reduce resistance received from a console device when an operator operates the console device. This console device is provided with: a gripped part that is gripped by an operator; a gimbal that rotatably supports the gripped part around a yaw axis, a pitch axis, and a roll axis that intersect each other; and a control unit that controls the gimbal. The gimbal includes: harness winding parts wound around respective joints around the yaw axis, around the pitch axis, and around the roll axis; drive units for applying torques to the respective joints; and angle sensors for detecting angles of the respective joints and outputting detection values of the angles of the respective joints to the control unit. The control unit controls the drive units on the basis of the detection values input from the angle sensors, thereby compensating the torques applied to the respective joints by the drive units by torsional torques of the respective joints caused by the harness winding parts.

## Description

### TECHNICAL FIELD

This disclosure relates to a console device.

### BACKGROUND ART

A manipulator, also known as an articulated robot, includes a plurality of links coupled in series. The motion of the manipulator is achieved by driving the joints using servomotors or the like.

A master-slave system is sometimes employed for remote control of the manipulator. In the master-slave system, when an operator manipulates a master console device at hand, the master console device transfers a control signal corresponding to the manipulation to a slave manipulator. The slave manipulator then follows the operation of the console device according to the control signal.

Torque due to the self-weight of the manipulator's links acts on the joints. Therefore, to accurately control the manipulator, a compensation technique is required to counteract such torque (see Patent Documents 1 to 3) .

Patent Document 1 discloses a technique for compensating for the self-weight of a link of a manipulator using a counterweight.

Patent Document 2 discloses a technique that uses both an actuator driving a joint of a manipulator and a self-weight compensation actuator. The self-weight compensation actuator applies torque to the joint to compensate for the self-weight of a link.

Patent Document 3 discloses a technique for setting a pressure target required for self-weight compensation based on the current position of a robot arm and executing pressure control of a pneumatic actuator based on the pressure target.

### CITATION LIST

### Patent Literature

Patent Document 1: JP 2021-130161 A
Patent Document 2: WO 2017/159188 A1
Patent Document 3: WO 2016/051495 A1

### SUMMARY OF INVENTION

### Technical Problem

In order to prevent the operator from feeling the weight of the console device when manipulating the console device by hand, a self-weight compensation technique is also required for the console device. However, while Patent Documents 1 to 3 disclose techniques related to self-weight compensation of manipulators, they do not disclose techniques related to self-weight compensation of console devices.

A console device has a control harness. This harness is routed from a proximal end of the console device, through a joint of the console device, to a distal end of the console device. The harness is wound around the joint to allow rotation of the joint. Such winding of the harness generates torque at the joint. This torque causes a sense of resistance for the operator manipulating the console device.

It is therefore an object of one or more embodiments of the present disclosure to reduce the resistance felt by the operator from the console device during manipulation of the console device.

### Solution to Problem

To solve the above problem, according to an aspect of the present disclosure, the console device comprises a grip, a gimbal and a controller. The grip is configured to be gripped by an operator. The gimbal rotatably supports the grip around a yaw axis, a pitch axis, and a roll axis, the yaw axis, the pitch axis, and the roll axis intersecting each other. The controller controls the gimbal. The gimbal includes harness windings, drivers, and angle sensors. The harness windings are wound around respective joints around the yaw axis, around the pitch axis, and around the roll axis. The drivers apply torque to the respective joints. The angle sensors detect angles of the respective joints and output detected values of the angles of the respective joints to the controller. The controller controls the drivers based on the detected values received from the respective angle sensors so as to compensate for torsional torque at the respective joints due to the respective harness windings with the torque applied to the respective joints by the drivers.

### Advantageous Effects of Invention

The console device of one or more embodiments of the present disclosure contributes to reducing the resistance felt by the operator from the console device during manipulation of the console device.

### BRIEF DESCRIPTION OF DRAWINGS

[FIG. 1] This is a block diagram illustrating a remote-controlled robot system.
[FIG. 2] This illustrates a console device.
[FIG. 3] This illustrates left and right consoles of the console device.
[FIG. 4] This illustrates a gimbal and a hand controller at a distal end of the right console.
[FIG. 5] This illustrates the gimbal and the hand controller at the distal end of the right console.
[FIG. 6] This is a graph showing a relationship between joint angle and torque.

### DESCRIPTION OF EMBODIMENTS

One or more embodiments of the present disclosure are described below with reference to the drawings. The features and technical effects of the embodiments are understood from the following detailed description and drawings. However, the scope of the invention is not limited to the embodiments disclosed below. The drawings are provided for illustrative purposes only, and the scope of the invention is not limited to the examples in the drawings.

### <1. Overview of Remote-controlled Robot System>

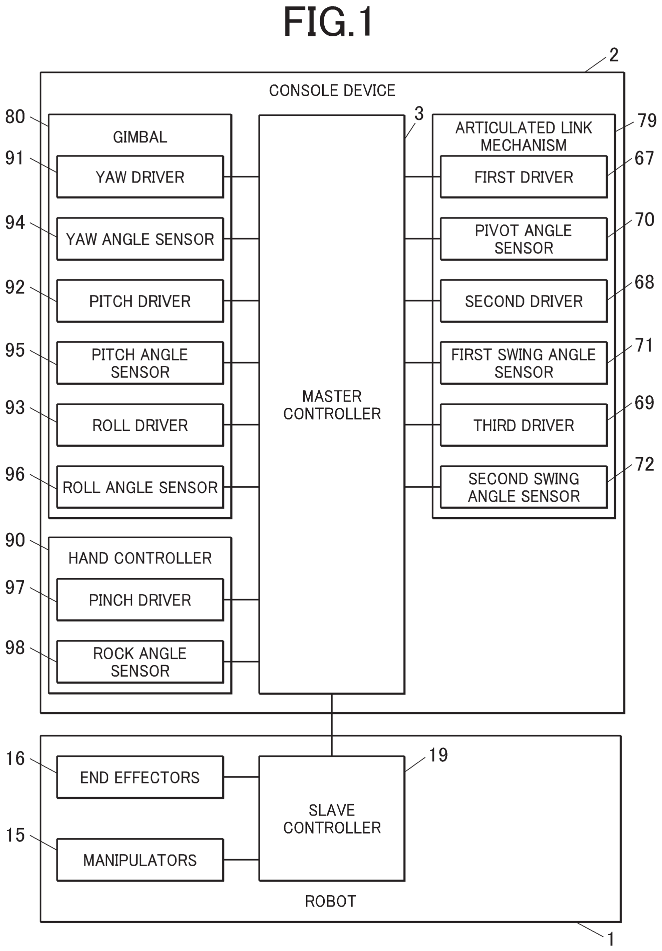

FIG. 1 is a block diagram illustrating a remote-controlled robot system.

The remote-controlled robot system includes a robot 1 and a console device 2. The console device 2 is a master and the robot 1 is a slave. When an operator, such as a doctor, manipulates the console device 2, the robot 1 operates in accordance with the operation of the console device 2.

### <2. Robot>

The robot 1 is installed in a workplace such as an operating room. The robot 1 is a robot that performs tasks such as surgery. The robot 1 includes two manipulators 15, two end effectors 16, and a slave controller 19.

The two manipulators 15 are disposed side by side on the left and right. Each of the manipulators 15 is a vertical articulated robot having five degrees of freedom, six degrees of freedom, or seven degrees of freedom. The manipulator 15 includes a plurality of links, a plurality of joints, and a plurality of drivers. These links are sequentially coupled in series by the joints from a proximal end to a distal end of the manipulator 15. These joints include bending and torsional joints as well as pivoting joints. The drivers are connected to the joints. The manipulator 15 is operated by the drivers applying torque to the joints.

Each of the end effectors 16 is coupled to the distal end of the manipulator 15. The end effector 16 may be a medical instrument such as forceps, tweezers, scissors, or a scalpel. In the present embodiment, the end effector 16 is forceps.

The slave controller 19 is one or more computers having a central processing unit (CPU), a random-access memory (RAM), a bus, a bus controller, an interface circuit, a drive circuit, a communication device, and the like. The slave controller 19 receives an operation signal from a master controller 3 of the console device 2. The slave controller 19 controls the manipulators 15 and end effectors 16 according to the operation signal, thereby causing the manipulators 15 and end effectors 16 to follow consoles 60 of the console device 2.

### <3. Console device>

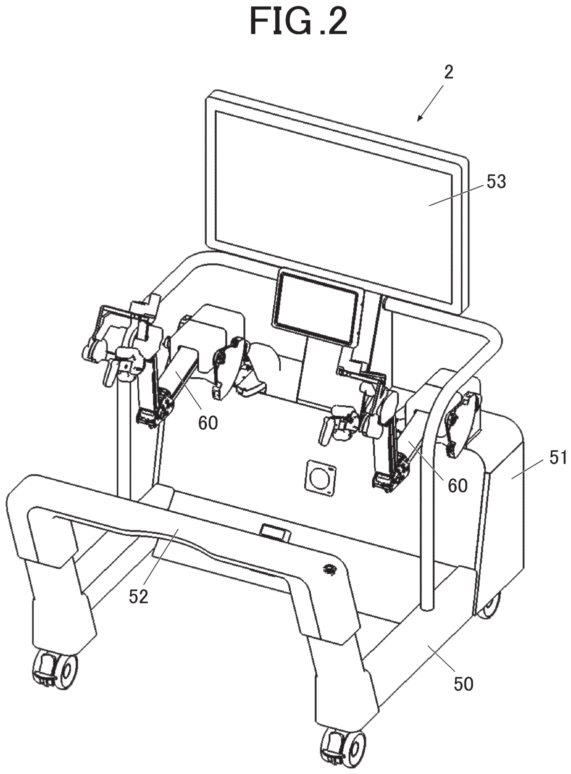

FIG. 2 is a perspective view of the console device 2.

The console device 2 is installed away from the workplace such as an operating room. The console device 2 may be installed in the workplace.

The console device 2 includes a cart 50, a base 51, a seat 52, a display 53, two consoles 60, and the master controller 3.

The master controller 3 is one or more computers having a central processing unit (CPU), a random-access memory (RAM), a bus, a bus controller, an interface circuit, a drive circuit, a communication device, and the like. The master controller 3 controls the console device 2. The master controller 3 can communicate with the slave controller 19 via a network or other means.

The cart 50 includes casters with stoppers on its underside and is movable by the casters.

The base 51 is fixed to the rear of the cart 50 and stands upright from the rear of the cart 50.

The seat 52 is fixed to the front of the cart 50 and stands upright from the front of the cart 50. The operator sits on the seat 52 and manipulates the consoles 60.

The display 53 is a full-color display, such as a liquid crystal display or an organic EL display. The display 53 displays images of the manipulator 15 and the end effector 16 captured by a camera.

The two consoles 60 are coupled to an upper portion of the base 51 and arranged side by side on the left and right with a gap between them. These consoles 60 extend forward from the base 51, allowing the operator to grasp the left and right consoles 60 with the left and right hands, respectively. When the operator moves the left console 60 with the left hand, the left manipulator 15 and left end effector 16 operate following the movement of the left console 60. When the operator moves the right console 60 with the right hand, the right manipulator 15 and right end effector 16 operate following the movement of the right console 60.

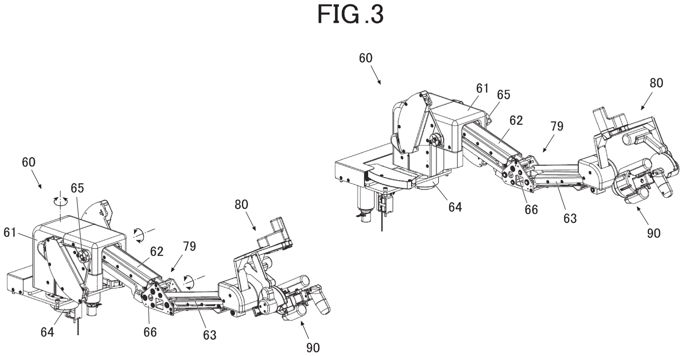

FIG. 3 is a perspective view of the left and right consoles 60.

As illustrated in FIG. 3, each of the consoles 60 includes an articulated link mechanism 79, a gimbal 80, and a grip 90.

A proximal end of the articulated link mechanism 79 is coupled to the base 51, a distal end of the articulated link mechanism 79 is coupled to the gimbal 80, and the grip 90 is coupled to the gimbal 80. Here, the term "distal" means farther from the base 51, and the term "proximal" means closer to the base 51.

The articulated link mechanism 79 supports the gimbal 80 and the grip 90 for translational movement with three degrees of freedom. The gimbal 80 rotatably supports the grip 90 with three degrees of freedom. When the operator grasps the grip 90 and moves the grip 90, the posture and orientation of the grip 90 is changed by the gimbal 80, and the grip 90 and gimbal 80 are translated by the articulated link mechanism 79. The articulated link mechanism 79 is utilized to adjust the position of the end effector 16 by the operator remotely manipulating the manipulator 15. The gimbal 80 is utilized to adjust the posture of the end effector 16 by the operator remotely manipulating the manipulator 15. The grip 90 is used for the operator to remotely manipulate the end effector 16. Therefore, hereinafter, the grip 90 is referred to as a hand controller 90.

The articulated link mechanism 79 includes a pivot 61, a proximal link 62, a distal link 63, a first joint 64, a second joint 65, and a third joint 66. As illustrated in FIG. 1, the articulated link mechanism 79 includes drivers 67 to 69 and sensors 70 to 72 that are utilized to control the articulated link mechanism 79.

The pivot 61 is rotatably coupled to an upper portion of the base 51 by the first joint 64. The pivot 61 is provided so as to pivot relative to the base 51 around a vertical pivot axis via the first joint 64. The pivot 61 and the first joint 64 correspond to the proximal end of the articulated link mechanism 79.

A pivot angle sensor 70, such as a rotary encoder, is provided on the base 51, and a first driver 67, such as a motor, is provided on the base 51. The pivot angle sensor 70 and the first driver 67 are coupled to the first joint 64. The pivot angle sensor 70 detects a pivot angle of the pivot 61 around the pivot axis and outputs the detected value to the master controller 3. The master controller 3 transfers the detected value of the pivot angle sensor 70 to the slave controller 19. The slave controller 19 controls the manipulator 15 according to the detected value of the pivot angle sensor 70.

The first driver 67 applies torque around the pivot axis to the first joint 64 and the pivot 61.

A proximal end of the proximal link 62 is rotatably coupled to the pivot 61 by the second joint 65. The proximal link 62 is provided so as to swing up and down relative to the pivot 61 around a horizontal first swing axis extending left and right via the second joint 65. The proximal link 62 may be configured by a link mechanism, such as a parallel link mechanism.

A first swing angle sensor 71, such as a rotary encoder, is provided in the pivot 61, and a second driver 68, such as a motor, is provided in the pivot 61. The second driver 68 and the first swing angle sensor 71 are coupled to the second joint 65. The first swing angle sensor 71 detects a first swing angle of the proximal link 62 around the first swing axis and outputs the detected value to the master controller 3. The master controller 3 transfers the detected value of the first swing angle sensor 71 to the slave controller 19. The slave controller 19 controls the manipulator 15 based on the detected value of the first swing angle sensor 71.

The second driver 68 applies torque around the first swing axis to the second joint 65 and the proximal link 62.

A proximal end of the distal link 63 is rotatably coupled to a distal end of the proximal link 62 by the third joint 66. The distal link 63 is provided so as to swing up and down relative to the proximal link 62 around a second swing axis parallel to the first swing axis via the third joint 66. The distal link 63 may be configured by a link mechanism, such as a parallel link mechanism.

A second swing angle sensor 72, such as a rotary encoder, is provided in the pivot 61, and a third driver 69, such as a motor, is provided in the pivot 61. The second swing angle sensor 72 and the third driver 69 are coupled to the third joint 66 via a link mechanism or other means. The second swing angle sensor 72 detects a second swing angle of the distal link 63 around the second swing axis and outputs the detected value to the master controller 3. The master controller 3 transfers the detected value of the second swing angle sensor 72 to the slave controller 19. The slave controller 19 controls the manipulator 15 based on the detected value of the second swing angle sensor 72.

The third driver 69 applies torque around the second swing axis to the third joint 66 and the distal link 63.

A distal end of the distal link 63 corresponds to the distal end of the articulated link mechanism 79, and the gimbal 80 is mounted on the distal end of the distal link 63.

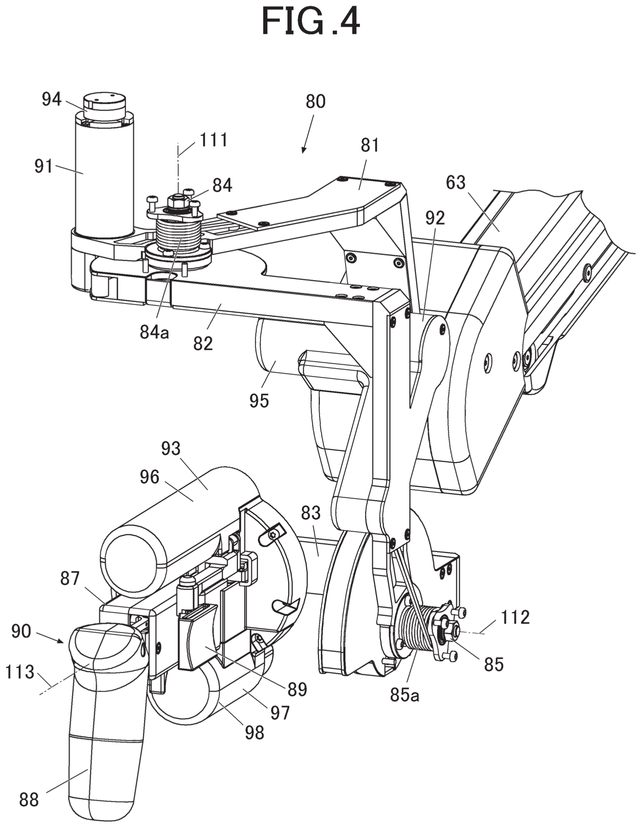

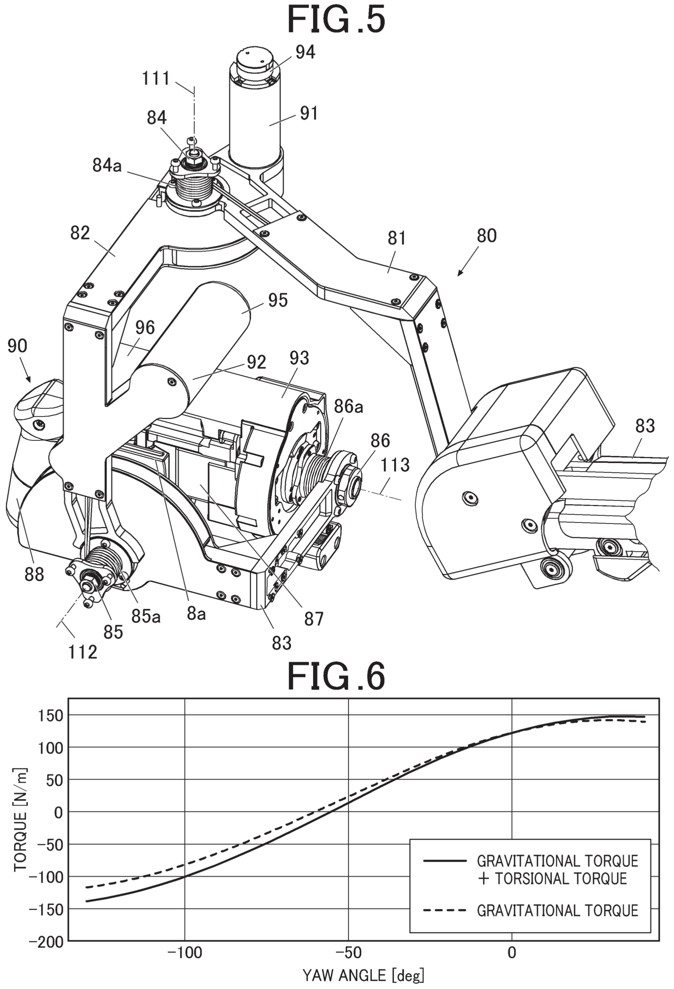

FIGs. 4 and 5 are perspective views of the right gimbal 80 and the right hand controller 90. The left gimbal 80 and the left hand controller 90 are bilaterally symmetrical to the right gimbal 80 and the right hand controller 90.

As illustrated in FIGs. 1, 4, and 5, the gimbal 80 includes a coupling arm 81, a first rotating arm 82, a second rotating arm 83, joints 84 to 86, harness windings 84a to 86a, drivers 91 to 93, and angle sensors 94 to 96.

A proximal end of the coupling arm 81 is mounted on the distal end of distal link 63. The coupling arm 81 forms an L-shaped bend from its proximal end to its distal end within the plane in which the distal link 63 swings up and down.

A proximal end of the first rotating arm 82 is rotatably coupled to the distal end of the coupling arm 81 by the joint 84. The first rotating arm 82 is rotatable relative to the coupling arm 81 around the yaw axis 111 of the joint 84. The yaw axis 111 is along the plane in which the distal link 63 swings up and down. The articulated link mechanism 79 supports the gimbal 80 and the hand controller 90 for translational movement. Therefore, the yaw axis 111 is vertical.

The harness winding 84a is wound around the joint 84. The harness winding 84a is formed by winding a bundle of wires running from the master controller 3 to the drivers 91 to 93, the angle sensors 94 to 96, a rock angle sensor 98 (described below), and a pinch driver 97 (described below). The harness winding 84a functions as a torsion spring. In other words, the harness winding 84a applies torsional torque around the yaw axis 111 to the joint 84. The torsional torque generated by the harness winding 84a is determined depending on the yaw angle of the first rotating arm 82 around the yaw axis 111. Specifically, the torsional torque is expressed as a linear function of the yaw angle. In other words, the torsional torque T_{y} is expressed by a formula "T_{y} = a_{y}θ_{y}+ b_{y}", where T_{y} is the torsional torque [Nm], θ_{y} is the yaw angle [deg], a_{y} is the torque spring constant of the harness winding 84a [Nm/deg], and b_{y} is the initial torsional torque [Nm] when the yaw angle is zero.

A yaw angle sensor 94, such as a rotary encoder, is coupled to the joint 84, and a yaw driver 91, such as a motor, is coupled to the joint 84. The yaw angle sensor 94 detects the yaw angle of the first rotating arm 82 around the yaw axis 111 and outputs the detected value to the master controller 3. The master controller 3 transfers the detected values of the yaw angle sensor 94 to the slave controller 19. The slave controller 19 controls the manipulator 15 based on the detected value of the yaw angle sensor 94.

The yaw driver 91 applies torque around the yaw axis 111 to the joint 84 and the first rotating arm 82.

A proximal end of the second rotating arm 83 is rotatably coupled to a distal end of the first rotating arm 82 by the joint 85. The second rotating arm 83 is rotatable relative to the first rotating arm 82 around the pitch axis 112 of the joint 85. The pitch axis 112 is orthogonal to the yaw axis 111. The articulated link mechanism 79 supports the gimbal 80 and the hand controller 90 for translational movement. Therefore, the pitch axis 112 is horizontal.

The harness winding 85a is wound around the joint 85. Similarly to the harness winding 84a, the harness winding 85a is formed by winding the bundle of wires. The harness winding 85a functions as a torsion spring that applies torsional torque around the pitch axis 112 to the joint 85. The torsional torque generated by the harness winding 85a is expressed as a linear function of the pitch angle of the second rotating arm 83 around the pitch axis 112.

A pitch angle sensor 95, such as a rotary encoder, is coupled to the joint 85, and a pitch driver 92, such as a motor, is coupled to the joint 85. The pitch angle sensor 95 detects the pitch angle of the first rotating arm 82 around the pitch axis 112 and outputs the detected value to the master controller 3. The master controller 3 transfers the detected value of the pitch angle sensor 95 to the slave controller 19. The slave controller 19 controls the manipulator 15 based on the detected value of the pitch angle sensor 95.

The pitch driver 92 applies torque around the pitch axis 112 to the joint 85 and the second rotating arm 83.

The hand controller 90 is rotatably coupled to a distal end of the second rotating arm 83 by the joint 86. The hand controller 90 is rotatable relative to the second rotating arm 83 around the roll axis 113 of the joint 86. The roll axis 113, pitch axis 112, and yaw axis 111 intersect each other at a common point of intersection.

The harness winding 86a is wound around the joint 86. Similarly to the harness winding 84a, the harness winding 86a is formed by winding the bundle of wires. The harness winding 86a functions as a torsion spring that applies torsional torque around the roll axis 113 to the joint 86. The torsional torque generated by the harness winding 86a is expressed as a linear function of the roll angle of the hand controller 90 around the roll axis 113.

A roll angle sensor 96, such as a rotary encoder, is coupled to the joint 86, and a roll driver 93, such as a motor, is coupled to the joint 86. The roll angle sensor 96 detects the roll angle of a tab 87 of the hand controller 90 around the roll axis 113 and outputs the detected value to the master controller 3. The master controller 3 transfers the detected values of the roll angle sensor 96 to the slave controller 19. The slave controller 19 controls the manipulator 15 based on the detected value of the roll angle sensor 96.

The roll driver 93 applies torque around the roll axis 113 to the joint 86 and the tab 87.

The hand controller 90 includes the tab 87, a handle 88, an operation lever 89, the pinch driver 97, and the rock angle sensor 98.

The tab 87 is rotatably coupled to the distal end of the second rotating arm 83 by the joint 86. The tab 87 is rotatable relative to the second rotating arm 83 around the roll axis 113 of the joint 86. The roll axis 113 is orthogonal to the pitch axis 112. The roll axis 113, pitch axis 112, and yaw axis 111 intersect each other at the common point of intersection. The tab 87 extends along the roll axis 113 from the joint 86 toward the common point of intersection.

The tab 87 is formed into a rectangular parallelepiped shape. The shape of the tab 87 may be cylindrical or elliptical with a central axis along the roll axis 113.

The operation lever 89 is disposed facing a side surface of the tab 87. A proximal end of the operation lever 89 is coupled to the tab 87 so as to be rotatable around an axis orthogonal to the roll axis 113 at a point closer to the joint 86. The operation lever 89 extends along the roll axis 113 from its proximal end to its distal end. The operation lever 89 is rockable around the axis at its proximal end so as to allow the operation lever 89 to be moved in and out of contact with the side surface of the tab 87. The operator rocks the operation lever 89, primarily with the index finger, to move the operation lever 89 in and out of contact with a first side surface 87a of the tab 87.

The pinch driver 97 includes, for example, a motor. The pinch driver 97 applies torque to the operation lever 89.

The rock angle sensor 98 includes, for example, a rotary encoder. The rock angle sensor 98 detects a rock angle of the operation lever 89 and outputs the detected value to the master controller 3. The master controller 3 transfers the detected value of the rock angle sensor 98 to the slave controller 19. The slave controller 19 controls the end effector 16 based on the detected value of the rock angle sensor 98.

The handle 88 is disposed facing an end surface of the tab 87. The handle 88 is mounted to the tab 87 via a linear guide. The handle 88 extends from the linear guide in a direction intersecting the roll axis 113. The handle 88 is shaped like a column, more specifically a cylinder, having a central axis intersecting the roll axis 113.

The handle 88 is movable along the roll axis 113 by the linear guide so as to allow the handle 88 to be moved in and out of contact with the end surface of the tab 87.

The operator places the palm of the hand on the handle 88, grasps the handle 88 with the palm, and pinches the tab 87 and the operation lever 89 with the fingers. When the operator holds the hand controller 90 in this manner, the burden on the operator's hand during manipulation of the hand controller 90 is reduced and the operator can manipulate the hand controller 90 with precision and finesse.

The handle 88 can be moved in and out of contact with the end surface of the tab 87. This enables the operator to adjust the position of the handle 88 according to the size of the hand.

The operator manipulates the pivot 61, proximal link 62, distal link 63, and gimbal 80 by moving the wrist, arm, shoulder, and upper body while grasping the handle 88. When the operator manipulates the pivot 61, proximal link 62, distal link 63, and gimbal 80, the master controller 3 transfers the detected values of the pivot angle sensor 70, first swing angle sensor 71, second swing angle sensor 72, yaw angle sensor 94, pitch angle sensor 95 and roll angle sensor 96 to the slave controller 19. The slave controller 19 controls the manipulator 15 based on the detected values of the pivot angle sensor 70, first swing angle sensor 71, second swing angle sensor 72, yaw angle sensor 94, pitch angle sensor 95 and roll angle sensor 96. This allows the left manipulator 15 to follow the operation of the left console 60 and the right manipulator 15 to follow the operation of the right console 60.

When the operator rocks the operation lever 89 by finger, the master controller 3 transfers the detected value of the rock angle sensor 98 to the slave controller 19. The slave controller 19 controls the end effector 16 based on the detected value of the rock angle sensor 98. This allows the left end effector 16 to follow the rocking of the operation lever 89 of the left hand controller 90 and the right end effector 16 to follow the operation of the operation lever 89 of the right hand controller 90.

### <4. Compensation>

To prevent the operator from receiving an excessive reaction force from the console 60 when the operator manipulates the console 60 while looking at the display 53, and to prevent the console 60 from moving due to its own weight and the torsional torque of the harness windings 84a, 85a, and 86a when the operator releases his/her hand from the console 60, the master controller 3 performs the following compensation processing.

### (1) Yaw Angle

### (1-1) Calculation of Counteracting Torque

As described above, the yaw angle sensor 94 detects the yaw angle of the first rotating arm 82 around the yaw axis 111 and outputs the detected value to the master controller 3. The master controller 3 calculates counteracting torque from the detected value of the yaw angle sensor 94. The counteracting torque is torque that counteracts both the gravitational torque generated at the joint 84 due to the weight of the first rotating arm 82 and the torsional torque generated at the joint 84 due to the torsion of the harness winding 84a. In other words, the counteracting torque is balanced by the sum of the gravitational torque and the torsional torque. FIG. 6 is a graph showing a relationship between the yaw angle and the torque. As indicated by the broken curve in FIG. 6, the gravitational torque is determined depending on the posture of the first rotating arm 82, that is, the yaw angle. The torsional torque of the harness winding 84a is determined depending on the yaw angle of the first rotating arm 82. Therefore, as indicated by the solid curve in FIG. 6, the sum of the gravitational torque and the torsional torque is determined depending on the yaw angle of the first rotating arm 82, and the counteracting torque that is balanced by the sum of the gravitational torque and the torsional torque is also determined depending on the yaw angle of the first rotating arm 82.

To calculate the counteracting torque, the master controller 3 pre-stores a function representing a relationship between the yaw angle of the first rotating arm 82 and the counteracting torque as a mathematical formula or a look-up table. Then, when the master controller 3 receives the detected value of the yaw angle from the yaw angle sensor 94, the master controller 3 applies the detected value to the function to calculate the counteracting torque corresponding to the detected value from the function. The function representing the relationship between the yaw angle of the first rotating arm 82 and the counteracting torque is obtained through a prior experiment or simulation.

### (1-2) Generation of Counteracting Torque

When the master controller 3 controls the yaw driver 91 to generate the counteracting torque, the yaw driver 91 generates the counteracting torque at the joint 84.

Thereafter, the master controller 3 executes the calculation process and the generation process of the counteracting torque every sampling cycle of the yaw angle sensor 94 or every integer multiple of the sampling cycle.

### (2) Pitch Angle

As described above, the pitch angle sensor 95 detects the pitch angle of the second rotating arm 83 around the pitch axis 112 and outputs the detected value to the master controller 3. The master controller 3 calculates counteracting torque from the detected value of the pitch angle sensor 95. Next, when the master controller 3 controls the pitch driver 92 to generate the counteracting torque, the pitch driver 92 generates the counteracting torque at the joint 85. Thereafter, the master controller 3 executes the calculation process and the generation process of the counteracting torque every sampling cycle of the pitch angle sensor 95 or every integer multiple of the sampling cycle.

### (3) Roll Angle

As described above, the roll angle sensor 96 detects the roll angle of the tab 87 of the hand controller 90 around the roll axis 113 and outputs the detected value to the master controller 3. The master controller 3 calculates counteracting torque from the detected value of the roll angle sensor 96. Next, when the master controller 3 controls the pitch driver 92 to generate the counteracting torque, the roll driver 93 generates the counteracting torque at the joint 86. Thereafter, the master controller 3 executes the calculation process and the generation process of the counteracting torque every sampling cycle of the roll angle sensor 96 or every integer multiple of the sampling cycle.

### <5. Summary>

The master controller 3 controls the yaw driver 91 based on the detected value received from the yaw angle sensor 94 so as to compensate for the torsional torque at the joint 84 due to the harness winding 84a with the torque applied to the joint 84 by the yaw driver 91. Specifically, the master controller 3 applies the detected value of the yaw angle sensor 94 to the function representing the relationship between the yaw angle of the joint 84 and the counteracting torque to calculate the counteracting torque corresponding to the detected value from the function. When the master controller 3 controls the yaw driver 91 to generate the counteracting torque, the yaw driver 91 generates the counteracting torque at the joint 84. The counteracting torque is balanced by the sum of the gravitational torque at the joint 84 due to the weight of the first rotating arm 82 and the torsional torque at the joint 84 due to the torsion of the harness winding 84a. Therefore, the operator is less likely to feel resistance around the yaw axis 111 when the operator moves the hand controller 90.

Similarly, the master controller 3 controls the pitch driver 92 based on the detected value received from the pitch angle sensor 95 so as to compensate for the torsional torque at the joint 85 due to the harness winding 85a with the torque applied to the joint 85 by the pitch driver 92. Therefore, the operator is less likely to feel resistance around the pitch axis 112 when the operator moves the hand controller 90.

Similarly, the master controller 3 controls the roll driver 93 based on the detected value received from the roll angle sensor 96 so as to compensate for the torsional torque at the joint 86 due to the harness winding 86a with the torque applied to the joint 86 by the roll driver 93. Therefore, the operator is less likely to feel resistance around the roll axis 113 when the operator moves the hand controller 90.

The reduced resistance felt by the operator from the hand controller 90 during manipulation of the hand controller 90 also reduces the sense of fatigue of the operator. The operator does not need to exert excessive force to manipulate the hand controller 90, enabling stable and safe remote manipulation of the robot 1.

### Reference Signs List

2 console device
3 master controller
80 gimbal
84 yaw axis joint
85 pitch axis joint
86 roll axis joint
91 yaw driver
92 pitch driver
93 roll driver
94 yaw angle sensor
95 pitch angle sensor
96 roll angle sensor
90 hand controller (grip)

## Claims

1. A console device comprising:
a grip configured to be gripped by an operator;
a gimbal that rotatably supports the grip around a yaw axis, a pitch axis, and a roll axis, the yaw axis, the pitch axis, and the roll axis intersecting each other; and
a controller that controls the gimbal,
wherein
the gimbal includes:
harness windings that are wound around respective joints around the yaw axis, around the pitch axis, and around the roll axis;
drivers that apply torque to the respective joints; and
angle sensors that detect angles of the respective joints and output detected values of the angles of the respective joints to the controller, and
wherein the controller controls the drivers based on the detected values received from the respective angle sensors to apply the torque to the respective joints in order to compensate for torsional torque at the respective joints due to the respective harness windings.

2. The console device according to claim 1, wherein the controller controls the drivers based on the detected values received from the respective angle sensors to cause the drivers to generate, at the respective joints, a counteracting torque that counteracts a gravitational torque at the respective joints due to gravity and that counteracts the torsional torque at the respective joints due to the respective harness windings.

3. The console device according to claim 2, wherein the controller executes:
a calculation process that calculates the counteracting torque from the detected values received from the respective angle sensors; and
a generation process that controls the drivers to generate, at the respective joints, the counteracting torque calculated in the calculation process.

4. The console device according to claim 3, wherein the controller executes the calculation process and the generation process each time the controller receives the detected values from the respective angle sensors.

5. The console device according to claim 3 or 4, wherein:
the controller pre-stores functions that represent respective relationships between the angles of the respective joints and the torque; and
the controller calculates, in the calculation process, the counteracting torque corresponding to the detected values received from the respective angle sensors from the respective functions.
